# EUROPÄISCHE PATENTANMELDUNG

(11) **EP 4 760 735 A1**
(43) Veröffentlichungstag der Anmeldung: **17.06.2026**
(21) Anmeldenummer: 25222394.6
(22) Anmeldetag: 10.12.2025
(51) Int. Cl.: G16H 10/60, G16H 30/20, G16H 30/40, G16H 40/20, G06F 21/62, G16H 40/67

(54) **SYSTEM ZUR ABSICHERUNG VON DATENSTRÖMEN IM MEDIZINISCHEN BEREICH**

(30) Priorität: 13.12.2024 DE 102024137573
(71) Anmelder: KARL STORZ SE & Co. KG, 78532 Baden-Württemberg Tuttlingen (DE)
(72) Erfinder: Brüderle, Klaus, 78532 Tuttlingen (DE)

(57) **Zusammenfassung**

Es werden ein System und ein Verfahren zur Absicherung von Datenströmen im medizinischen Bereich, die durch Transportkomponenten geleitet werden, bereitgestellt. Das System umfasst: eine Erfassungseinrichtung, welche dazu eingerichtet ist, Daten eines Datenstroms von mindestens einer Transportkomponente zu erfassen; eine Ermittlungseinrichtung, welche dazu eingerichtet ist, basierend auf den erfassten Daten, Informationen über einen Bearbeitungsschritt des Datenstromes zu ermitteln; eine Texterzeugungseinrichtung, welche dazu eingerichtet ist, eine Beschreibung des ermittelten Bearbeitungsschritts zu erzeugen; eine Auswerteeinrichtung, welche dazu eingerichtet ist, festzustellen, ob der beschriebene Bearbeitungsschritt in einer Liste vorbestimmter Bearbeitungsschritte enthalten ist; und eine Ausgabeschnittstelle, welche dazu eingerichtet ist, ein Ausgabesignal mit Informationen zumindest über die in dem Datenstrom ausgeführten Bearbeitungsschritte aus der Liste der vorbestimmten Bearbeitungsschritte auszugeben.

## Beschreibung

### Technisches Gebiet der Erfindung

Die vorliegende Erfindung betrifft ein System und ein Verfahren zur Absicherung von Datenströmen im medizinischen Bereich, vorzugsweise von Datenströmen, die einer Anonymisierung bedürfen.

### Hintergrund der Erfindung

In medizinischen Prozeduren und Eingriffen werden üblicherweise Datenströme an einer Quelle, beispielsweise der Kamera eines Endoskops, erzeugt und über ein oder mehrere zusammengeschaltete Kommunikationssysteme bis zum Endbenutzer geleitet. Das Signal wird von den verschiedenen Komponenten des Kommunikationssystems entlang des Signalwegs transportiert und verarbeitet.

Die Daten erfahren somit an vielen Stellen Bearbeitungsschritte, welche über das einfache Weiterleiten des ursprünglichen Signals hinausgehen. Beispielsweise können die Daten umformatiert, komprimiert, oder zusammengefasst werden.

In der Regel wird das Signal auch System- oder Netzwerkgrenzen überschreiten. Beispielsweise kann ein Bildstrom in einem Operationssaal erzeugt, über das Krankenhausnetzwerk transportiert und dann außerhalb des Krankenhausnetzwerks an das Internet oder ein Cloud-Archiv weitergeleitet werden.

Die Datenströme müssen den geltenden Sicherheitsanforderungen und Datenschutzbestimmungen entsprechen. Dies gilt insbesondere für Komponenten, die sich an Systemgrenzen befinden. Bevor etwa ein Portalserver oder ein Bilddrucker Daten weiterleitet, muss sichergestellt sein, dass keine Sicherheitsprobleme vorliegen.

Derzeit sind Datenströme, je nach Kommunikationssystem und Ausführung, mittels einer Authentifizierung (z. B. einer Zugangssicherung durch eine Benutzerprüfung) und/oder eine Autorisierung, welche den Berechtigungen eines Benutzers entspricht, gesichert. Die Daten und Transportkanäle sind in der Regel verschlüsselt, so dass authentifizierte und autorisierte Benutzer mit gesicherten Daten und Transportkanälen arbeiten können.

Mit diesen Methoden kann eine Transportkomponente, wie etwa ein Portalserver oder ein Router, den Transportkanal sichern. Die derzeit üblichen Sicherungsmaßnahmen sichern jedoch nur den Transportkanal. Eine Transportkomponente, die einen Bearbeitungsschritt überprüfen muss (z.B. das Vorhandensein einer Anonymisierung in einem Datenstrom), muss die Bearbeitungsschritte nachverfolgen. Dies bedeutet im Prinzip, dass alle Transportkomponenten, die einen Datenstrom transportiert haben, kontaktiert werden müssen. Dies kann sehr zeit- und kostenintensiv sein.

Es ist daher wichtig, Vorrichtungen und Verfahren zu entwickeln, welche den Zugriff auf Informationen über die an einem Datenstrom von den Transportkomponenten eines Kommunikationssystems durchgeführten Bearbeitungsschritte vereinfachen, insbesondere um die Erfüllung von Sicherheitsanforderungen zu erleichtern.

### Zusammenfassung der Erfindung

Es ist daher eine Aufgabe der vorliegenden Erfindung, ein System und ein Verfahren bereitzustellen, die es ermöglichen, die von verschiedenen Transportkomponenten an einem Datenstrom durchgeführten Bearbeitungsschritte mit minimalem Aufwand zu verfolgen.

Diese Aufgabe wird durch die Gegenstände der unabhängigen Patentansprüche der vorliegenden Erfindung gelöst.

Gemäß einem ersten Aspekt wird demnach ein System zur Absicherung von Datenströmen, insbesondere im medizinischen Bereich, die durch Transportkomponenten geleitet werden, bereitgestellt. Das System umfasst eine Erfassungseinrichtung, welche dazu eingerichtet ist, Daten eines Datenstroms von mindestens einer Transportkomponente zu erfassen; eine Ermittlungseinrichtung, welche dazu eingerichtet ist, basierend auf den erfassten Daten Informationen über einen Bearbeitungsschritt des Datenstroms zu ermitteln; eine Texterzeugungseinrichtung, welche dazu eingerichtet ist, eine Beschreibung des ermittelten Bearbeitungsschritts zu erzeugen; eine Auswerteeinrichtung, welche dazu eingerichtet ist, festzustellen, ob der beschriebene Bearbeitungsschritt in einer Liste vorbestimmter Bearbeitungsschritte enthalten ist; und eine Ausgabeschnittstelle, welche dazu eingerichtet ist, ein Ausgabesignal mit Informationen zumindest über die in dem Datenstrom ausgeführten Bearbeitungsschritte aus der Liste der vorbestimmten Bearbeitungsschritte auszugeben.

Eine grundlegende Idee der vorliegenden Erfindung besteht darin, eine Absicherung von Datenströmen durch die Ermittlung der verschiedenen Bearbeitungsschritte, die durch ein Kommunikationssystem von den Transportkomponenten durchgeführt werden, zu schaffen. Nach dem Stand der Technik kann eine solche Absicherung nur durch die Kommunikation von Transportkomponenten mit anderen Transportkomponenten erreicht werden. Nach dem ersten Aspekt der vorliegenden Erfindung wird eine Dokumentation der Bearbeitungsschritte durchgeführt, so dass eine Transportkomponente in der dokumentierten Kette von Bearbeitungsschritte u.a. verifizieren kann, ob gewisse Absicherungsmaßnahmen bereits übernommen sind, ohne mit anderen Transportkomponenten zu kommunizieren. Somit wird eine Historie der durchgeführten Bearbeitungen erzeugt und bereitgestellt. Damit können sicherheitsrelevante Aspekte des Bildstrominhalts verifiziert werden, beispielweise ob persönliche Daten anonymisiert worden sind. Somit kann eine Absicherung des Inhaltes von Medienströmen und deren Bearbeitung geschaffen werden.

Ein Vorteil der vorliegenden Erfindung besteht darin, dass Transportkomponenten in einem Kommunikationssystem nicht isoliert oder atomar agieren, sondern anhand des erfindungsgemäßen Systems einen Überblick über alle ausgeführten Bearbeitungsschritte haben können. Beispielsweise kann ein Portalserver, welcher einen OP-Datenstrom in ein öffentliches Netz überträgt, in Echtzeit prüfen, ob Personengesichter anonymisiert worden sind. Dies erfolgt, ohne Bilder aus dem OP-Datenstrom selbst zu untersuchen oder Informationen mit anderen Transportkomponenten auszutauschen, sondern unter Zuhilfenahme der Beschreibung der durchgeführten Bearbeitungsschritte.

Damit wird es für die Transportkomponenten (oder Bearbeitungskomponenten) möglich, eigene Bearbeitungsschritte durchzuführen, unter Berücksichtigung der Vorgeschichte des Inhaltes des Datenstroms auf dem Signalpfad. Ohne das erfindungsgemäße System müsste sich eine Bearbeitungskomponente über zusätzliche Kommunikationskanäle bei den vorhergehenden Bearbeitungskomponenten über die durchgeführten Bearbeitungen informieren.

Vorteilhaft kann das System des ersten Aspekts der Erfindung für alle Arten von medizinischen Prozeduren verwendet werden, und alle Arten von Datenquellen.

Die Daten, welche die Erfassungseinrichtung erfassen kann, können hier alle Datenformate umfassen, die in einem Kommunikationssystem übertragen werden können. Die Daten können beispielsweise Bilddaten (die von einer externen Bildgebungseinrichtung übertragen werden) oder Textdaten umfassen. Es kann sich um Rohdaten oder bereits vorverarbeitete Daten handeln. Bei den durch eine Bildgebungseinrichtung erfassten Bilddaten kann es sich insbesondere um ein Bild oder mehrere Bilder handeln, insbesondere eine zeitliche Abfolge von Bildern.

Transportkomponenten sind als funktionale Teile eines Kommunikationssystems zu verstehen, d.h. als Einheiten, die für die Ausführung einer Funktion ausgelegt sind. Diese Funktion umfasst in der Regel den Transport eines Signals, kann aber auch verschiedene Signalverarbeitungsvorgänge umfassen. Im Folgenden werden Transportkomponenten und Bearbeitungskomponenten synonym verwendet. Eine Transportkomponente kann als Software und/oder Hardware realisiert werden. Als Hardware kann eine Transportkomponente eine Vorrichtung, ein Teil einer Vorrichtung oder eine Anzahl von zusammenverbundenen Vorrichtungen sein.

Die für einen Bearbeitungsschritt ermittelten Informationen sind weit zu fassen. Dazu gehört mindestens eine Identifizierung der Art und des Umfangs des Bearbeitungsschritts. Die Informationen können auch eine Identifizierung der Bearbeitungskomponente umfassen. Vorzugsweise wird auch der Zeitpunkt einer Bearbeitung in die Informationen aufgenommen, damit eine Chronologie der Bearbeitungsschritte erstellt werden kann.

Eine Beschreibung der ermittelten Bearbeitungsschritts ist als Text zu verstehen, aber auch als kodierte Informationen, welche von Transportkomponenten interpretiert werden können (z.B. Maschinencode). Die Beschreibung kann in einem Ausgabesignal codiert werden, so dass die Beschreibung über das Kommunikationssystem von Komponente zu Komponente übertragen werden kann.

Eine Liste vorbestimmter Bearbeitungsschritte kann insbesondere Bearbeitungsschritte enthalten, die Sicherheitsanforderungen erfüllen. Das Ausgangssignal der Ausgangsschnittstelle kann somit Informationen darüber liefern, ob alle Sicherheitsanforderungen erfüllt sind, um einen Datenstrom auf dem Signalpfad weiterleiten zu können.

Obwohl hier, im Vorstehenden und im Folgenden einige Funktionen als von "Einrichtungen" oder "Schnittstellen" ausgeführt beschrieben werden, versteht es sich, dass dies nicht unbedingt bedeutet, dass solche Einrichtungen, oder Schnittstellen als voneinander getrennte Vorrichtungen bereitgestellt werden. In Fällen, in denen eine oder mehrere Einrichtungen oder Schnittstellen ganz oder teilweise als Software bereitgestellt werden, können die Einrichtungen oder Schnittstellen durch Programmcodeabschnitte oder Programmcodeschnipsel implementiert werden, die sich voneinander unterscheiden, aber auch miteinander verwoben sein können.

In ähnlicher Weise können in dem Fall, in dem ein oder mehrere Einrichtungen oder Schnittstellen als Hardware bereitgestellt werden, die Funktionen einer oder mehrerer Einrichtungen oder Schnittstellen von ein und derselben Hardwarekomponente bereitgestellt werden, oder die Funktionen einer Einrichtung oder einer Schnittstelle, oder die Funktionen mehrerer Einrichtungen oder Schnittstellen können auf mehrere Hardwarekomponenten verteilt sein, die nicht notwendigerweise den Einrichtungen oder Schnittstellen eins zu eins entsprechen müssen. Daher ist jede Vorrichtung, jedes System, jedes Verfahren usw., das alle Merkmale und Funktionen aufweist, die einer bestimmten Einrichtung und/oder einer bestimmten Schnittstelle zugeschrieben werden, so zu verstehen, dass sie bzw. es die Einrichtung und/oder die Schnittstelle ausbildet, umfasst, oder implementiert.

Insbesondere besteht die Möglichkeit, dass einige oder alle Einrichtungen und Schnittstelle durch ausführbare Programmcode implementiert werden.

Insbesondere können die Ermittlungseinrichtung, die Texterzeugungseinrichtung und die Auswerteeinrichtung als jede Vorrichtung oder jedes Mittel zum Rechnen realisiert werden, insbesondere zum Ausführen einer Software, einer App oder eines Algorithmus. Beispielsweise können die Ermittlungseinrichtung und die Texterzeugungseinrichtung mindestens einen Prozessor, wie etwa mindestens einen Zentralprozessor, CPU und/oder mindestens einen Grafikprozessor, GPU, und/oder mindestens einem Tensorprozessor, TPU, und/oder mindestens einem Datenprozessor, DPU, und/oder mindestens ein feldprogrammierbares Gate-Array, FPGA, und/oder mindestens einen anwendungsspezifischen integrierten Schaltkreis, ASIC, und/oder eine beliebige Kombination der Vorstehenden umfassen. Die Ermittlungseinrichtung und/oder die Texterzeugungseinrichtung und/oder die Auswerteeinrichtung können ferner einen Arbeitsspeicher aufweisen, der mit dem mindestens einen Prozessor operativ verbunden ist, und/oder einen nicht-flüchtigen Speicher, der mit dem mindestens einen Prozessor und/oder dem Arbeitsspeicher operativ verbunden ist. Die Ermittlungseinrichtung, die Texterzeugungseinrichtung und die Auswerteeinrichtung können teilweise und/oder vollständig in einer lokalen Vorrichtung und/oder teilweise und/oder vollständig in einem entfernten System, wie z. B. durch eine Cloud-Computing-Plattform, implementiert sein.

Die Ermittlungseinrichtung und/oder die Texterzeugungseinrichtung und/oder die Auswerteeinrichtung können auch mit Maschinenlernen-Modellen ausgestattet sein. Die Ermittlungseinrichtung und die Auswerteeinrichtung können dazu eingerichtet sein, eine Erkennung von Bearbeitungsschritten mit Maschinenlernen-Modellen durchzuführen. Die Texterzeugungseinrichtung kann mit einem oder mehreren großen Sprachmodellen (auf Englisch, large language models, LLM) ausgestattet sein, die eine Beschreibung der erkannten Bearbeitungsschritte erzeugen.

Die Erfassungseinrichtung kann dazu eingerichtet sein, Bilddaten als den Datenstrom oder im Zuge des Datenstroms direkt von einer Bildgebungseinrichtung zu erhalten, insbesondere in Echtzeit, oder alternativ auch von einem Bildablage- und Kommunikationssystem (PACS, von engl. "picture archiving and communications system"), wobei auch letzteres in Echtzeit erfolgen kann, also insbesondere sobald die Bilddaten in dem PACS eingehen. Das System kann auch eine Bildgebungseinrichtung umfassen, sodass das Erhalten der Bilddaten auch ein Erfassen der Bilddaten durch die Bildgebungseinrichtung umfassen kann. Das System kann mehrere verschiedene Bildgebungseinrichtungen umfassen, welche beispielsweise für das Erfassen von Daten in unterschiedlichen Formaten ausgebildet sein können. Die Verarbeitung von den Daten kann jedoch stets mit derselben Ermittlungseinrichtung erfolgen.

Die Ausgabeschnittstelle kann eine Benutzerschnittstelle umfassen oder an einer Benutzerschnittstelle oder an einen Monitor verbunden sein. Somit kann das Ausgabesignal für einen Nutzer zur Verfügung stehen.

Weitere Vorteile der Erfindung werden im Folgenden anhand der Gegenstände der Unteransprüche und insbesondere anhand der Beschreibung der Figuren erläutert.

Gemäß einigen bevorzugten Ausführungsformen, Varianten oder Verfeinerungen von Ausführungsformen umfassen die Daten Bildgebungsdaten einer Kamera eines Endoskops. Die Daten können somit Bilder (Fotoaufnahmen oder Videoaufnahmen) einer endoskopischen Prozedur umfassen. Erfindungsgemäß werden die von den verschiedenen Transportkomponenten durchgeführten Bearbeitungsschritte inhaltlich beschrieben. Dies liefert wichtige Information für einen Endnutzer. Zum Beispiel kann ein Chirurg, der die Bilder analysiert und eine Diagnose stellen möchte, schnell feststellen, welchen Bearbeitungen die Bilder unterworfen wurden. Der Nutzer kann auch fehlende Bearbeitungen identifizieren und dafür sorgen, dass diese ausgeführt werden. Beispielsweise kann ein Chirurg feststellen, dass eine Anonymisierung fehlt, und die Anonymisierung durch die entsprechende Bearbeitungskomponente verlangen, etwa mittels der Benutzerschnittstelle.

Gemäß einigen bevorzugten Ausführungsformen, Varianten oder Verfeinerungen von Ausführungsformen ist die Texterzeugungseinrichtung ferner dazu eingerichtet, die Beschreibung des ermittelten Bearbeitungsschritts eines Datenstroms als Metadaten zu erzeugen und die Beschreibung dem Datenstrom hinzuzufügen. Das erfindungsgemäße System kann die Beschreibung der Bearbeitungsschritte laufend aktualisieren, wobei die aktualisierte Beschreibung vorteilhaft in dem Datenstrom implementiert oder in den Datenstrom integriert wird. Die Beschreibung der Bearbeitungsschritte ist bei diesen Ausführungsformen inhaltlicher Bestandteil des Datenstroms. Die Transportkomponenten können somit auf die Chronologie der Bearbeitungsschritte zugreifen, ohne mit dem erfindungsgemäßen System kommunizieren zu müssen.

Gemäß einigen bevorzugten Ausführungsformen, Varianten oder Verfeinerungen von Ausführungsformen umfasst das System eine Datenbank, welche dazu eingerichtet ist, die erzeugte Beschreibung des ermittelten Bearbeitungsschritts zu speichern, und für die Transportkomponenten und/oder über die Ausgabeschnittstelle abrufbar zur Verfügung zu stellen. In einigen Ausführungsformen der Erfindung wird die Beschreibung der Bearbeitungsschritte für die verschiedenen Datenströme zentral gespeichert. Beispielsweise kann in jeden Datenstrom eine Adresse eingefügt werden, die Informationen darüber enthält, wo die Beschreibung der Bearbeitungsschritte in der Datenbank gespeichert ist. Damit ist die Chronologie der Bearbeitungsschritte zugänglich, muss aber nicht unbedingt selbst in dem Datenstrom enthalten sein. Sie kann bei Bedarf (und entsprechender Berechtigung) abgerufen werden. Transportkomponenten, die keine Informationen über die Vorgeschichte der Bearbeitungsschritte benötigen, können darauf verzichten.

Gemäß einigen bevorzugten Ausführungsformen, Varianten oder Verfeinerungen von Ausführungsformen umfasst die Beschreibung des Bearbeitungsschritts mindestens eines der folgenden: Erzeugungsdatum eines Inhalts; welche Transportkomponente den Bearbeitungsschritt durchgeführt hat; Art und/oder Umfang des durchgeführten Bearbeitungsschritts. Die Beschreibung offenbart vorteilhaft jeden datentechnischen Aspekt eines Bearbeitungsschritts, welchen eine Transportkomponente im Signalpfad durchführt. Mindestanforderungen sind dabei der Zeitpunkt der Bearbeitung (um eine Chronologie der Bearbeitungen erstellen zu können) und eine Zuordnung der Bearbeitung zu der Bearbeitungskomponente (typischerweise eine der Transportkomponenten).

Gemäß einigen bevorzugten Ausführungsformen, Varianten oder Verfeinerungen von Ausführungsformen umfasst die Art der durchgeführten Bearbeitung eine Umformatierung der Datenströme, eine Kombinierung von Datenströmen, oder eine Anonymisierung der Datenströme. Die Art der Bearbeitung beschreibt welche Bearbeitung durchgeführt wurde. Wie oben erwähnt, kann die Ermittlungseinrichtung ein (oder mehrere) Maschinenlernen-Modell verwenden, um die Bearbeitungen durch Klassifizierung zu erkennen. Beispielsweise kann ein trainierter Kl-Algorithmus dazu eingesetzt werden, um Bearbeitungen zu klassifizieren. Alternativ oder zusätzlich kann die Ermittlungseinrichtung die Art einer Bearbeitung von der entsprechenden Bearbeitungskomponente erfahren, beispielsweise über einen Code.

Gemäß einigen bevorzugten Ausführungsformen, Varianten oder Verfeinerungen von Ausführungsformen ist die Beschreibung als ein oder mittels eines oder über einen QR-Code codiert. Der QR-Code kann sich in dem Datenstrom befinden, während die Beschreibung in einem Zentralserver oder in einer Cloud-Plattform gespeichert sein könnte, wobei die Beschreibung dort durch den QR-Code erreichbar ist. Die Beschreibung kann auch nicht sichtbar (d.h. steganographisch) in dem Bildstrom angeordnet sein. Alternativ könnte die Beschreibung außerhalb des Bildinhalts stehen, beispielsweise in einer Hilfsdatei.

Gemäß einigen bevorzugten Ausführungsformen, Varianten oder Verfeinerungen von Ausführungsformen ist die Ausgabeschnittstelle dazu ausgelegt, das Ausgabesignal an eine Transportkomponente zu übertragen. Die Beschreibung eines Bearbeitungsschritts kann somit an eine bestimmte Transportkomponente übermittelt werden, insbesondere an eine Transportkomponente, die eine Bearbeitung des Datenstroms durchführen soll. Bei diesen Ausführungsformen kann die Transportkomponente die Beschreibung der bereits durchgeführten Bearbeitungsschritte parallel zu dem Datenstrom erhalten. Die Ausgabeschnittstelle kann so ausgelegt sein, dass sie nach Anforderung durch eine Transportkomponente ein Ausgabesignal mit der Historie der Verarbeitungsschritte ausgibt.

Gemäß einigen bevorzugten Ausführungsformen, Varianten oder Verfeinerungen von Ausführungsformen ist die Texterzeugungseinrichtung dazu ausgelegt, die Beschreibung des Bearbeitungsschritts in mindestens einem, in mehreren, oder in jedem Frame der Daten zu notieren. Wenn die Notierung bildweise erfolgt, d.h. Bild für Bild, können die Frames einzeln aussortiert werden. Dies ist vorteilhaft, wenn einige Bearbeitungsschritte nur bestimmte Frames eines Bildstroms betreffen. Wie oben erwähnt, kann die Beschreibung der Bearbeitungsschritte als QR-Code codiert werden. Dabei ist es denkbar, dass jeder Frame mit einem einzelnen QR-Code gekennzeichnet wird.

Gemäß einigen bevorzugten Ausführungsformen, Varianten oder Verfeinerungen von Ausführungsformen ist die Ausgabeschnittstelle ferner dazu ausgelegt, ein Warnungssignal auszugeben, wenn aus der Liste der vorbestimmten Bearbeitungsschritte nicht alle Bearbeitungsschritte ausgeführt worden sind. Die aufgelisteten vorbestimmten Bearbeitungsschritte umfassen insbesondere Sicherheitsanforderungen, die erfüllt werden sollen, beispielsweise eine Anonymisierung des Datenstroms. Wenn die fehlende Ausführung der Bearbeitungsschritte gegen Sicherheitsanforderungen verstößt, darf eine Transportkomponente den Datenstrom nicht weiterleiten. Bei Vorliegen des Warnungssignals erkennt eine Transportkomponente, ohne den Datenstrom prüfen zu müssen, dass der Datenstrom nicht ohne weiteres weitergeleitet werden darf. Das Warnungssignal kann in Maschinensprache und/oder als Text ausgegeben werden, um einen Benutzer zu informieren.

Gemäß einigen bevorzugten Ausführungsformen, Varianten oder Verfeinerungen von Ausführungsformen ist die Erfassungseinrichtung dazu ausgelegt, Daten von mindestens einer der folgenden (Arten von) Transportkomponenten zu erfassen: einem Portalserver, einem Router, einem Archiv, einer Anzeigeeinheit, einem Drucker, einem Encoder, einem Formatkonverter, einem Datenkompressor, und/oder einem Datenkombinierer. Der Umfang der Erfindung beschränkt sich nicht auf bestimmte Transportkomponenten. Das erfindungsgemäße System ist zur Anwendung bei beliebigen Transportkomponenten geeignet.

Gemäß einem zweiten Aspekt stellt die vorliegende Erfindung ein computerimplementiertes Verfahren zur Absicherung von Datenströmen im medizinischen Bereich, die durch Transportkomponenten geleitet werden, bereit, mindestens umfassend die Schritte: Erfassen von Daten eines Datenstroms von zumindest einer Transportkomponente; Ermitteln, basierend auf den erfassten Daten, von Informationen über einen Bearbeitungsschritt des Datenstroms; Erzeugen einer Beschreibung des ermittelten Bearbeitungsschritts; Feststellen, ob der beschriebene Bearbeitungsschritt in einer Liste vorbestimmter Bearbeitungsschritte enthalten ist; und Ausgeben eines Ausgabesignals, mit Informationen zumindest über die in dem Datenstrom ausgeführten Bearbeitungsschritte aus der Liste der vorbestimmten Bearbeitungsschritte.

Das computerimplementierte Verfahren des zweiten Aspekts kann vorzugsweise mit dem System des ersten Aspekts ausgeführt werden. In ähnlicher Weise können die verschiedenen Ausführungsformen des Systems gemäß Ausführungsformen des computerimplementierten Verfahrens des zweiten Aspekts betrieben werden.

Gemäß einem dritten Aspekt stellt die vorliegende Erfindung ein Datenübertragungssystem bereit, umfassend eine Bildgebungseinrichtung, eine Mehrzahl von Transportkomponenten und das System gemäß dem ersten Aspekt der Erfindung, wobei die Bildgebungseinrichtung dazu ausgelegt ist, mindestens einen Datenstrom an mindestens eine von der Mehrzahl von Transportkomponenten zu übermitteln, wobei die Mehrzahl von Transportkomponenten eingerichtet sind, Bearbeitungsschritte auf den Datenstrom durchzuführen, und wobei das System gemäß dem ersten Aspekt der Erfindung dazu ausgelegt ist, Daten über die von der mindestens einen Transportkomponente der Mehrzahl von Transportkomponenten auf den Datenstrom ausgeführten Bearbeitungsschritte zu erfassen.

Gemäß einem vierten Aspekt stellt die Erfindung ein Computerprogrammprodukt bereit, das ausführbaren Programmcode umfasst, welcher dazu eingerichtet ist, wenn er von einer Recheneinrichtung ausgeführt wird, dass Verfahren gemäß einer Ausführungsform des zweiten Aspekts der vorliegenden Erfindung durchzuführen.

Gemäß einem fünften Aspekt stellt die Erfindung ein nicht-flüchtiges, computerlesbares Datenspeichermedium bereit, das ausführbaren Programmcode umfasst, welcher dazu eingerichtet ist, wenn er von einer Recheneinrichtung ausgeführt wird, dass Verfahren gemäß einer Ausführungsform des zweiten Aspekts der vorliegenden Erfindung durchzuführen.

Das nicht-flüchtige, computerlesbare Datenspeichermedium kann jede Art von Computerspeicher, insbesondere Halbleiterspeicher, wie z.B. einen Festkörperspeicher, umfassen oder aus einem solchen bestehen. Das Datenträger kann auch eine CD, eine DVD, eine Blu-Ray-Disc, einen USB-Speicherstick oder dergleichen umfassen oder daraus bestehen.

Gemäß einem sechsten Aspekt stellt die Erfindung einen Datenstrom bereit, der ausführbaren Programmcode umfasst oder zum Erzeugen von ausführbarem Programmcode konfiguriert ist, welcher dazu eingerichtet ist, wenn er von einer Recheneinrichtung ausgeführt wird, dass Verfahren gemäß einer Ausführungsform des zweiten Aspekts der vorliegenden Erfindung durchzuführen.

Weitere vorteilhafte Varianten, Optionen, Ausführungsformen und Modifikationen ergeben sich aus den folgenden Figuren, der ausführlichen Beschreibung, sowie aus den Ansprüchen. Es versteht sich jedoch, dass die detaillierte Beschreibung und die spezifischen Beispiele zwar bevorzugte Ausführungsformen der Erfindung angeben, aber nur zur Veranschaulichung angeführt werden, da verschiedene Änderungen und Modifikationen innerhalb des Umfangs der Erfindung für den Fachmann ersichtlich sind.

### Kurzbeschreibung der Figuren

Einzelne Ausführungsformen der vorliegenden Offenbarung werden anhand der folgenden Abbildungen im Detail erläutert werden. Die Bestandteile in den Zeichnungen sind nicht notwendigerweise maßstabsgetreu, sondern dienen dazu, die Grundsätze der vorliegenden Erfindung zu veranschaulichen. Teile in den verschiedenen Abbildungen, die den gleichen Elementen oder Verfahrensschritten entsprechen, wurden in den Abbildungen mit den gleichen Bezugszeichen versehen. Die Nummerierung von Verfahrensschritten dient zunächst nur zu deren Unterscheidung und impliziert nicht zwingend eine entsprechende Reihenfolge, wobei es jedoch eine Variante darstellt, die Schritte in der Reihenfolge ihrer Nummerierung durchzuführen. Mehrere Schritte können auch überlappend oder gleichzeitig durchgeführt werden. Von den Figuren zeigen:
- Fig. 1: ein schematisches Blockdiagramm zum Erläutern eines Systems gemäß einer Ausführungsform des ersten Aspekts der vorliegenden Erfindung;
- Fig. 2: ein schematisches Flussdiagramm zur Erläuterung eines computerimplementierten Verfahrens gemäß einer Ausführungsform des zweiten Aspekts der vorliegenden Erfindung;
- Fig. 3: ein schematisches Blockdiagramm zur Erläuterung eines Datenübertragungssystems gemäß einer Ausführungsform des dritten Aspekts der vorliegenden Erfindung;
- Fig. 4: ein schematisches Blockdiagramm eines Computerprogrammprodukts gemäß einer Ausführungsform des vierten Aspekts der vorliegenden Erfindung; und
- Fig. 5: ein schematisches Blockdiagramm eines nicht-flüchtigen computerlesbaren Datenspeichermediums gemäß einer Ausführungsform des fünften Aspekts der vorliegenden Erfindung.

### Detaillierte Beschreibung der Figuren

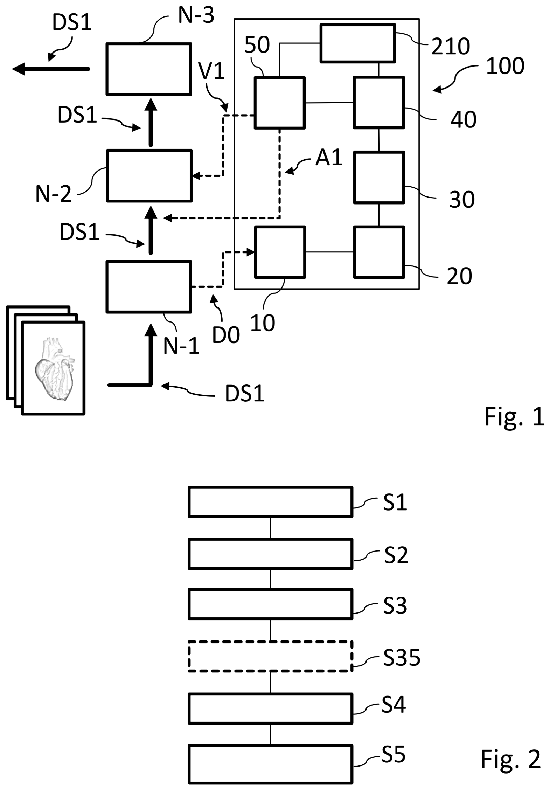

Fig. 1 zeigt ein schematisches Blockdiagramm zum Erläutern eines Systems 100 zur Absicherung von Datenströmen, insbesondere im medizinischen Bereich, die durch Transportkomponenten N1, N2, N3 (im Nachfolgenden teilweise zusammenfassend als N-i bezeichnet) geleitet werden. Das in Fig. 1 dargestellte erfindungsgemäße System 100 umfasst eine Erfassungseinrichtung 10, eine Ermittlungseinrichtung 20, eine Texterzeugungseinrichtung 30, eine Auswerteeinrichtung 40 und eine Ausgabeschnittstelle 50.

In der Fig. 1 sind Bildgebungsdaten dargestellt, die beispielsweise von einer Bildgebungseinrichtung (etwa der Kamera eines Endoskops) erzeugt werden können. Diese Bildgebungsdaten werden als Datenstrom DS1 durch die Transportkomponenten N-1, N-2 und N-3 transportiert. Die Transportkomponenten N-i können Einheiten eines Kommunikationsnetzwerks, beispielsweise eines Krankenhausnetzes sein. In der Fig. 1 ist der Einfachheit halber ein Kommunikationsnetz mit drei Transportkomponenten N-1, N-2 und N-3 dargestellt. Die Transportkomponenten N-i können beispielsweise einen Portalserver, einen Router, ein Archiv, eine Anzeigeeinheit, einen Drucker, einen Encoder, einen Formatkonverter, einen Datenkompressor, oder einen Datenkombinierer sein oder umfassen. Einige Transportkomponenten N-i sind intern verbunden, d.h. nur mit anderen Transportkomponenten N-i des Kommunikationsnetzes verbunden. In der Fig. 1 sind die Transportkomponenten N-1 und N-2 intern verbunden. Die Transportkomponente N-1 kann mit einer Bildgebungseinrichtung (nicht in Fig. 1 dargestellt) verbunden sein, welche in der Regel Teil des Krankenhausnetzes ist. Andere Transportkomponenten N-i sind auch mit externen Einheiten verbunden. Die Transportkomponente N-3 könnte beispielsweise ein Portalserver sein, der den Datenstrom DS1 an einen Drucker, ein Archiv, einen Server oder eine Cloud-Plattform (nicht in Fig. 1 dargestellt) übertragen kann bzw. überträgt.

Die Erfassungseinrichtung 10 des Systems 100 ist dazu eingerichtet, Daten D0 von mindestens einer Transportkomponente N-1 zu erfassen. Die Erfassungseinrichtung 10 kann in Hardware und/oder Software implementiert werden und kann insbesondere Signalen von den Transportkomponenten N-i empfangen und/oder an die Transportkomponenten N-i übertragen. Die Erfassungseinrichtung 10 kann vorzugsweise eine Programmierschnittstelle (engl. "application programming interface", API) enthalten, damit eine beidseitige Kommunikation mit den Transportkomponenten N-i erstellt werden könnte.

Die Ermittlungseinrichtung 20 ist dazu eingerichtet, auf Basis der erfassten Daten D0 Informationen über einen Bearbeitungsschritt des Datenstroms DS1 zu ermitteln. In Fig. 1 kann der Bearbeitungsschritt beispielsweise von der Transportkomponente N-1 durchgeführt und dann von der Ermittlungseinrichtung 20 erkannt werden. Die Ermittlungseinrichtung 20 kann jede Vorrichtung oder jedes Mittel zum Rechnen sein, insbesondere zum Ausführen einer Software, einer App oder eines Algorithmus.

Die Ermittlungseinrichtung 20 kann zusätzlich mit mindestens einem Maschinenlernen-Modell ausgestattet werden, um eine Erkennung von Bearbeitungsschritten durchzuführen. Die Erkennung eines Bearbeitungsschritts kann auch von einer Transportkomponente N-i veranlasst werden. In einigen Ausführungsformen der Erfindung können beispielsweise die Transportkomponenten N-i das System 100 der Erfindung über die durchgeführten Bearbeitungsschritte unaufgefordert informieren. In Fig. 1 kann beispielsweise die Transportkomponente N-1 die Erfassungseinrichtung 10 über den von ihr durchgeführten Bearbeitungsschritt informieren.

Die Texterzeugungseinrichtung 30 ist dazu ausgebildet, eine Beschreibung des ermittelten Bearbeitungsschritts des Datenstroms DS1 bereitzustellen. Diese Beschreibung kann in Form von Metadaten dem Datenstrom hinzugefügt werden. Die Beschreibung der Bearbeitungsschritte ist bei diesen Ausführungsformen inhaltlicher Bestandteil des Datenstroms DS1. Die Texterzeugungseinrichtung 30 kann mit einem oder mehreren Maschinenlernen-Modellen, beispielweise mit einem oder mehreren großen Sprachmodellen (LLM) ausgestattet werden, die eine Beschreibung der erkannten Bearbeitungsschritte erstellen und/oder unterstützen können.

Die Beschreibung eines Bearbeitungsschritts ist vorteilhaft so detailliert, dass die Art und/oder der Umfang des Bearbeitungsschritts eindeutig sind. Mindestanforderungen sind dabei der Zeitpunkt der Bearbeitung (Erzeugungsdatum eines Inhalts), um eine Chronologie der Bearbeitungen erstellen zu können, und eine Zuordnung der Transportkomponente N-i zu den durchgeführten Bearbeitungen. Die Art einer durchgeführten Bearbeitung kann beispielsweise eine Umformatierung der Datenströme DS1, eine Kombinierung von Datenströmen DS1, oder eine Anonymisierung der Datenströme DS1 sein oder umfassen.

Die Beschreibung kann auch als QR-Code codiert sein. Der QR-Code kann in dem Datenstrom integriert oder beinhaltet sein, während die Beschreibung in einem Zentralserver oder in einer Cloud-Plattform angeordnet sein kann, wobei die Beschreibung durch den QR-Code abrufbar ist. Die Beschreibung könnte auch steganographisch in dem Bildstrom angeordnet sein, oder außerhalb des Bildinhalts stehen, beispielsweise in einer Hilfsdatei.

Die Texterzeugungseinrichtung 30 kann ferner dazu ausgelegt sein, die Beschreibung des Bearbeitungsschritts in jedem Frame der Daten D0 zu notieren. Die Transportkomponenten N-i können somit auf die Chronologie der Bearbeitungsschritte zugreifen, ohne mit dem erfindungsgemäßen System 100 kommunizieren zu müssen.

Die Auswerteeinrichtung 40 ist dazu eingerichtet, festzustellen, ob der beschriebene Bearbeitungsschritt in einer Liste vorbestimmter Bearbeitungsschritte enthalten ist. Diese Liste wird vorzugsweise Bearbeitungsschritte umfassen, die mit Sicherheitsanforderungen zu tun haben, insbesondere Sicherheitsanforderungen erfüllen und/oder durchsetzen. Ein solcher Bearbeitungsschritt kann etwa eine Anonymisierung sein. Um einen Bearbeitungsschritt als einen Bearbeitungsschritt zu identifizieren, der in der Liste enthalten ist, kann die Auswerteeinrichtung 40 ein oder mehrere Maschinenlernen-Modelle umfassen, die für die Klassifizierung von Bearbeitungsschritten trainiert sind.

Das System 100 umfasst ferner eine Ausgabeschnittstelle 50, welche dazu eingerichtet ist, ein Ausgabesignal A1 mit Informationen zumindest über die in dem Datenstrom DS1 ausgeführten Bearbeitungsschritte aus der Liste der vorbestimmten Bearbeitungsschritte auszugeben. Das Ausgabesignal A1 kann an eine Transportkomponente N-i übertragen werden, insbesondere an eine Transportkomponente N-2 (wie in Fig. 1 dargestellt), die eine Bearbeitung des Datenstroms DS1 nach dem durch die Transportkomponente N-1 ausgeführten Bearbeitungsschritt durchführen soll.

Die Ausgabeschnittstelle 50 kann dazu ausgebildet sein, nach einer Anforderung durch eine Transportkomponente N-i (beispielsweise die Transportkomponente N-2) ein Ausgabesignal A1 mit der Historie der Bearbeitungsschritte auszugeben. Bei diesen Ausführungsformen kann die Transportkomponente N-2 die Beschreibung der Bearbeitungsschritte parallel zu dem Datenstrom DS1 erhalten. Alternativ oder zusätzlich kann das Ausgabesignal A1 dem Datenstrom DS1 hinzugefügt werden (wie in Fig. 1 dargestellt).

Die Ausgabeschnittstelle 50 kann ferner dazu ausgelegt sein, ein Warnungssignal V1 auszugeben, wenn aus der Liste der vorbestimmten Bearbeitungsschritte nicht alle Bearbeitungsschritte ausgeführt worden sind. Die aufgelisteten vorbestimmten Bearbeitungsschritte umfassen in der Regel Sicherheitsanforderungen, die erfüllt werden sollen, beispielsweise eine Anonymisierung des Datenstroms DS1.

Werden die nicht ausgeführten Bearbeitungsschritte gegen Sicherheitsanforderungen verstoßen, darf eine Transportkomponente N-i den Datenstrom nicht weiterleiten. Bei Vorliegen des Warnungssignals V1 erkennt eine Transportkomponente N-i, ohne den Datenstrom DS1 prüfen zu müssen, dass der Datenstrom DS1 nicht ohne weiteres weitergeleitet werden darf. Das Warnungssignal V1 kann in Maschinensprache und/oder als Text ausgegeben werden, um einen Benutzer die Information zu verdeutlichen oder zu vermitteln.

Die Ausgabeschnittstelle 50 kann eine Benutzerschnittstelle umfassen oder mit einer Benutzerschnittstelle oder mit einem Monitor verbunden sein. Somit kann das Ausgabesignal A1 und /oder das Warnungssignal V1 für einen Nutzer sichtbar zur Verfügung stehen.

Das in Fig. 1 dargestellte System 100 umfasst ferner eine Datenbank 210, welche dazu eingerichtet ist, die erzeugte Beschreibung der ermittelten Bearbeitungsschritte zu speichern, und für die Transportkomponenten N-i und/oder über die Ausgabeschnittstelle 50 abrufbar zur Verfügung zu stellen. Damit kann die Beschreibung der Bearbeitungsschritte für die verschiedenen Datenströme DS1 zentral gespeichert werden. Beispielsweise kann in jeden Datenstrom DS1 eine Verlinkung oder eine Adresse eingeschrieben werden, die Informationen darüber enthält, wo die Beschreibung der Bearbeitungsschritte in der Datenbank 210 gespeichert ist.

Das erfindungsgemäße System 100 stellt somit eine durchgängige, aktualisierte und vollständige Vorgeschichte der durchgeführten Bearbeitungsschritte bereit, welche die verschiedenen Transportkomponenten an Datenströmen DS1, insbesondere Medienströmen entlang eines Signalpfads ausgeführt haben. Dabei werden sämtliche Bearbeitungsschritte der an dem Signalpfad beteiligten Transportkomponenten N-i erfasst und die entsprechenden Informationen fortlaufend als Bestandteil des Inhalts des Datenstroms DS1 eingetragen.

Fig. 2 zeigt ein schematisches Flussdiagramm zum Erläutern eines Verfahrens gemäß einer Ausführungsform des zweiten Aspekts der vorliegenden Erfindung, d.h., eines computerimplementierten Verfahrens zur Absicherung von Datenströmen DS1, insbesondere im medizinischen Bereich. Das Verfahren gemäß Fig. 2 ist insbesondere mittels des Systems 100 aus Fig. 1 durchführbar und kann daher gemäß allen in Bezug auf das erfindungsgemäße System 100 beschriebenen Optionen oder Varianten angepasst werden und umgekehrt.

In einem Schritt S1 werden Daten D0 von zumindest einer Transportkomponente N-i erfasst. Die Daten D0 können beispielsweise Bilder sein, die von der Kamera eines Endoskops empfangen werden oder wurden.

In einem Schritt S2 werden, auf Basis der erfassten Daten D0, Informationen über mindestens einen Bearbeitungsschritt (vorzugsweise mehrere, oder alle Bearbeitungsschritte) eines Datenstroms DS1 ermittelt.

In einem weiteren Schritt S3 wird eine Beschreibung des ermittelten Bearbeitungsschritts erzeugt. In einem optionalen Schritt S35 kann diese Beschreibung vorzugsweise Teil des Inhalts des Datenstroms DS1 sein oder gemacht werden.

In einem Schritt S4 wird festgestellt, ob der beschriebene Bearbeitungsschritt in einer Liste vorbestimmter Bearbeitungsschritte enthalten ist. Diese Liste von vorbestimmten Bearbeitungsschritten enthält vorzugsweise Sicherheitsanforderungen, die der Datenstrom DS1 erfüllen muss, beispielsweise eine Anonymisierung von persönlichen Daten, insbesondere solchen, anhand derer eine Person identifizierbar ist, z.B. eine Anonymisierung von Personengesichtern.

In einem Schritt S5 wird ein Ausgabesignal A1 ausgegeben, welche Informationen zumindest über die in dem Datenstrom DS1 ausgeführten Bearbeitungsschritte aus der Liste der vorbestimmten Bearbeitungsschritte enthält.

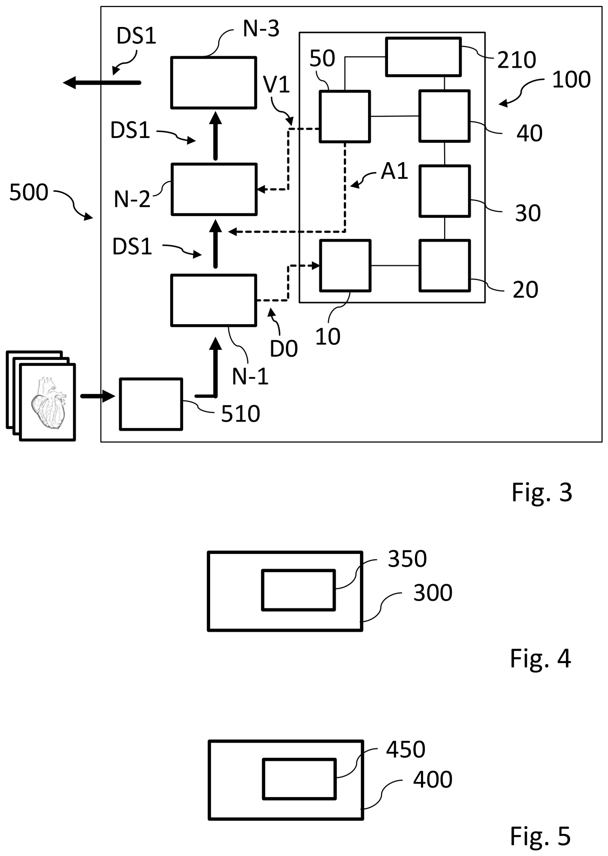

Fig. 3 zeigt ein schematisches Blockdiagramm zur Erläuterung eines Datenübertragungssystems 500, umfassend eine Bildgebungseinrichtung 510, eine Vielzahl von Transportkomponenten N-i und das System 100 gemäß einer Ausführungsform des ersten Aspekts der Erfindung.

Die Bildgebungseinrichtung 510 kann beispielweise die Kamera eines Endoskops sein. Die Bildgebungseinrichtung 510 erzeugt Bildgebungsdaten als ein Datenstrom DS1, der an mindestens eine Transportkomponente N-1 von einer Mehrzahl von Transportkomponenten N-i übertragen wird. Die verschiedenen Transportkomponenten N-i leiten den Datenstrom DS1 weiter und/oder führen Bearbeitungen (Bearbeitungsschritte) aus.

Wenn ein Bearbeitungsschritt von einer Transportkomponente N-i durchgeführt wird, erfasst das System 100 die entsprechende Information, erkennt den Bearbeitungsschritt, erstellt eine Beschreibung, die vorzugsweise dem Datenstrom DS1 hinzufügt wird, und gibt ein Ausgabesignal A1 aus, wie es im Vorangehenden in Bezug auf Fig. 1 beschrieben wird.

Fig. 4 zeigt ein schematisches Blockdiagramm eines Computerprogrammprodukts 300 gemäß einer Ausführungsform des dritten Aspekts der vorliegenden Erfindung. Das Computerprogrammprodukt 300 umfasst ausführbaren Programmcode 350, welcher dazu eingerichtet ist, wenn er ausgeführt wird (z.B. durch eine Recheneinrichtung), das Verfahren gemäß einer Ausführungsform der vorliegenden Erfindung durchzuführen, beispielsweise gemäß Fig. 2.

Fig. 5 zeigt ein schematisches Blockdiagramm eines nicht-flüchtigen computerlesbaren Datenspeichermediums 400 gemäß einer Ausführungsform der vorliegenden Erfindung. Das Datenspeichermedium 400 umfasst ausführbaren Programmcode 450, welcher dazu eingerichtet ist, wenn er ausgeführt wird (z.B. durch eine Recheneinrichtung), das Verfahren gemäß einer Ausführungsform des zweiten Aspekts der vorliegenden Erfindung durchzuführen, beispielsweise gemäß Fig. 2.

Das nicht-flüchtige computerlesbare Datenspeichermedium 400 kann beispielsweise als ein Halbleiterspeicher, z.B. ein SSD-Speicherstein ausgebildet sein oder einen solchen aufweisen. Das Datenspeichermedium 400 kann auch eine CD, DVD, Blu-Ray oder eine magnetische Speichervorrichtung aufweisen oder umfassen.

Die vorstehende Beschreibung der offenbarten Ausführungsformen enthält lediglich Beispiele für mögliche Umsetzungen, die beschrieben werden, um einen Fachmann in die Lage zu versetzen, die vorliegende Erfindung herzustellen oder verwenden zu können. Verschiedene Variationen und Modifikationen dieser Ausführungsformen sind für den Fachmann - nach Kenntnis der vorliegenden Erfindung - leicht ersichtlich, und die hierin definierten allgemeinen Prinzipien können auf andere Ausführungsformen angewendet werden, ohne vom Umfang der vorliegenden Offenbarung abzuweichen.

Somit soll die vorliegende Erfindung nicht auf die hierin gezeigten, spezifischen Ausführungsformen beschränkt sein, sondern ihr soll der breiteste Umfang zugestanden werden, der mit den hierin offenbarten Prinzipien und Merkmalen übereinstimmt.

### Bezugszeichenliste

- 10: Erfassungseinrichtung
- 20: Ermittlungseinrichtung
- 30: Texterzeugungseinrichtung
- 40: Auswerteeinrichtung
- 50: Ausgabeschnittstelle
- 100: System
- 210: Datenbank
- 300: Computerprogrammprodukt
- 350: Programmcode
- 400: Datenspeichermedium
- 450: Programmcode
- 500: Datenübertragungssystem
- 510: Bildgebungseinrichtung
- A1: Ausgabesignal
- D0: Daten
- DS1: Datenstrom
- N-i: Transportkomponenten
- S1...S35: Verfahrensschritte
- V1: Warnungssignal

## Patentansprüche

1. System (100) zur Absicherung von Datenströmen (DS1) im medizinischen Bereich, die durch Transportkomponenten (N-i) geleitet werden, umfassend:
eine Erfassungseinrichtung (10), welche dazu eingerichtet ist, Daten (D0) eines Datenstroms (DS1) von mindestens einer Transportkomponente (N-i) zu erfassen;
eine Ermittlungseinrichtung (20), welche dazu eingerichtet ist, basierend auf den erfassten Daten (D0), Informationen über einen Bearbeitungsschritt des Datenstroms (DS1) zu ermitteln;
eine Texterzeugungseinrichtung (30), welche dazu eingerichtet ist, eine Beschreibung des ermittelten Bearbeitungsschritts zu erzeugen;
eine Auswerteeinrichtung (40), welche dazu eingerichtet ist, festzustellen, ob der beschriebene Bearbeitungsschritt in einer Liste vorbestimmter Bearbeitungsschritte enthalten ist; und
eine Ausgabeschnittstelle (50), welche dazu eingerichtet ist, ein Ausgabesignal (A1) mit Informationen zumindest über die in dem Datenstrom (DS1) ausgeführten Bearbeitungsschritte aus der Liste der vorbestimmten Bearbeitungsschritte auszugeben.

2. System (100) nach Anspruch 1,
wobei die Daten (D0) Bildgebungsdaten einer Kamera eines Endoskops sind oder umfassen.

3. System (100) nach einem der Ansprüche 1 oder 2,
wobei die Texterzeugungseinrichtung (30) ferner dazu eingerichtet ist, die Beschreibung des ermittelten Bearbeitungsschritts eines Datenstroms (DS1) als Metadaten zu erzeugen und die Beschreibung dem Datenstrom (DS1) hinzuzufügen.

4. System (100) nach einem der Ansprüche 1 bis 3,
ferner umfassend eine Datenbank (210), welche dazu eingerichtet ist, die erzeugte Beschreibung des ermittelten Bearbeitungsschritts zu speichern, und für die Transportkomponenten (N-i) und/oder über die Ausgabeschnittstelle (50) abrufbar zur Verfügung zu stellen.

5. System (100) nach einem der Ansprüche 1 bis 4,
wobei die Beschreibung des Bearbeitungsschritts mindestens eines der folgenden umfasst:
Erzeugungsdatum eines Inhalts;
Transportkomponente (N-i), welche den Bearbeitungsschritt durchgeführt hat; und/oder
Art des durchgeführten Bearbeitungsschritts.

6. System (100) nach Anspruch 5,
wobei die Art des durchgeführten Bearbeitungsschritts eine Umformatierung der Datenströme (DS1), eine Kombinierung von Datenströmen (DS1), oder eine Anonymisierung der Datenströme (DS1) ist oder umfasst.

7. System (100) nach einem der Ansprüche 1 bis 6,
wobei die Beschreibung als QR-Code codiert oder über einen QR-Code verlinkt ist.

8. System (100) nach einem der Ansprüche 1 bis 7,
wobei die Ausgabeschnittstelle (50) dazu ausgelegt ist, das Ausgabesignal (A1) an mindestens eine der Transportkomponenten (N-i) zu übertragen.

9. System (100) nach einem der Ansprüche 1 bis 8,
wobei die Texterzeugungseinrichtung (30) dazu ausgelegt ist, die Beschreibung des Bearbeitungsschritts in jedem Frame der Daten (D0) zu notieren.

10. System (100) nach einem der Ansprüche 1 bis 9,
wobei die Ausgabeschnittstelle (50) ferner dazu ausgelegt ist, ein Warnungssignal (V1) auszugeben, wenn aus der Liste der vorbestimmten Bearbeitungsschritte nicht alle die Bearbeitungsschritte ausgeführt sind.

11. System (100) nach einem der Ansprüche 1 bis 10,
wobei die Erfassungseinrichtung (10) dazu ausgelegt ist, Daten (D0) von den folgenden Transportkomponenten (N-i) zu erfassen: einem Portalserver, einem Router, einem Archiv, einer Anzeigeeinheit, einem Drucker, einem Encoder, einem Formatkonverter, einem Datenkompressor, oder einem Datenkombinierer.

12. Computerimplementiertes Verfahren zur Absicherung von Datenströmen (DS1) im medizinischen Bereich, die durch Transportkomponenten (N-i) geleitet werden, mindestens umfassend die Schritte:
Erfassen (S1) von Daten (D0) eines Datenstroms (DS1) von zumindest einer Transportkomponente (N-i);
Ermitteln (S2), basierend auf den erfassten Daten (D0), von Informationen über einen Bearbeitungsschritt des Datenstroms (DS1);
Erzeugen (S3) einer Beschreibung des ermittelten Bearbeitungsschritts;
Feststellen (S4), ob der beschriebene Bearbeitungsschritt in einer Liste vorbestimmter Bearbeitungsschritte enthalten ist; und
Ausgeben (S5) eines Ausgabesignals (A1), mit Informationen zumindest über die in dem Datenstrom (DS1) ausgeführten Bearbeitungsschritte aus der Liste der vorbestimmten Bearbeitungsschritte.

13. Datenübertragungssystem (500), umfassend
eine Bildgebungseinrichtung (510), eine Mehrzahl von Transportkomponenten (N-i) und das System (100) nach einem der Ansprüche 1 bis 10,
wobei die Bildgebungseinrichtung (510) dazu ausgelegt ist, mindestens ein Datenstrom (DS1) an mindestens eine von der Mehrzahl von Transportkomponenten (N-i) zu übermitteln,
wobei die Transportkomponenten der Mehrzahl von Transportkomponenten (N-i) eingerichtet sind, Bearbeitungsschritte auf den Datenstrom (DS1) durchzuführen, und
wobei das System (100) nach einem der Ansprüche 1 bis 10 dazu ausgelegt ist, Daten (D0) über die von der mindestens einen der Mehrzahl von Transportkomponenten (N-i) auf den Datenstrom (DS1) ausgeführten Bearbeitungsschritte zu erfassen.

14. Computerprogrammprodukt (300), umfassend ausführbaren Programmcode (350), welcher dazu eingerichtet ist, wenn er ausgeführt wird, das Verfahren gemäß Anspruch 12 durchzuführen.

15. Nicht-flüchtiges, computerlesbares Datenspeichermedium (400), umfassend ausführbaren Programmcode (450), welcher dazu eingerichtet ist, wenn er ausgeführt wird, das Verfahren gemäß Anspruch 12 durchzuführen.
